# EUROPEAN PATENT SPECIFICATION

(11) **EP 2 297 304 B1**
(45) Date of publication and mention of the grant of the patent: **13.09.2017**
(21) Application number: 09742132.5
(22) Date of filing: 07.05.2009
(51) Int. Cl.: C12N 5/077, A61K 35/32

(54) **HUMAN BONE-FORMING CELLS IN THE TREATMENT OF CONDITIONS AND BONE DISEASES ASSOCIATED WITH IMMUNODEFICIENCY OR IMMUNOSUPPRESSION**
ZELLEN ZUR FORMUNG MENSCHLICHER KNOCHEN BEI DER BEHANDLUNG VON IMMUNSCHWÄCHE- ODER IMMUNSUPRESSION-ASSOZIIERTEN LEIDEN UND KNOCHENERKRANKUNGEN
CELLULES DE FORMATION D'OS HUMAINS POUR LE TRAITEMENT DE CONDITIONS ET MALADIES OSSEUSES ASSOCIÉES À L'IMMUNODÉPRESSION OU L'IMMUNOSUPPRESSION

(30) Priority: 07.05.2008 EP 08155768
(43) Date of publication of application: 23.03.2011
(73) Proprietor: Bone Therapeutics S.A., 6041 Gosselies (BE)
(72) Inventor: ALBARANI, Valentina, B-1160 Brussels (BE); BASTIANELLI, Enrico, B-1640 Rhode-Saint-Genèse (BE); BADOER, Cindy, 1460 Ittre (BE); PESESSE, Xavier, B-1430 Bierghes (BE)
(74) Representative: Michalík, Andrej
(86) International application number: PCT/EP2009/055559
(87) International publication number: WO 2009/135914

(56) References cited:
- WO-A-2007/093431
- KIM SEOK-JUNG ET AL: "Treatment of osteonecrosis of the femoral head using autologous cultured osteoblasts: a case report." JOURNAL OF MEDICAL CASE REPORTS 2008, vol. 2, 25 February 2008 (2008-02-25), page 58, XP021040524 ISSN: 1752-1947
- LE BLANC KATARINA ET AL: "HLA expression and immunologic properties of differentiated and undifferentiated mesenchymal stem cells." EXPERIMENTAL HEMATOLOGY OCT 2003, vol. 31, no. 10, October 2003 (2003-10), pages 890-896, XP002422938 ISSN: 0301-472X cited in the application
- NIEMEYER PHILIPP ET AL: "Comparison of immunological properties of bone marrow stromal cells and adipose tissue-derived stem cells before and after osteogenic differentiation in vitro." TISSUE ENGINEERING JAN 2007, vol. 13, no. 1, January 2007 (2007-01), pages 111-121, XP002496955 ISSN: 1076-3279
- LIU HUA ET AL: "The immunogenicity and immunomodulatory function of osteogenic cells differentiated from mesenchymal stem cells." JOURNAL OF IMMUNOLOGY (BALTIMORE, MD. : 1950) 1 MAR 2006, vol. 176, no. 5, 1 March 2006 (2006-03-01), pages 2864-2871, XP002476155 ISSN: 0022-1767
- SKJODT H ET AL: "Human osteoblast-like cells expressing MHC class II determinants stimulate allogeneic and autologous peripheral blood mononuclear cells and function as antigen-presenting cells." IMMUNOLOGY NOV 1989, vol. 68, no. 3, November 1989 (1989-11), pages 416-420, XP002496956 ISSN: 0019-2805 cited in the application
- GANGJI ET AL: "Treatment of osteonecrosis of the femoral head with implantation of preosteoblastic cells into the necrotic zone: Case report", BONE, PERGAMON PRESS., OXFORD, GB, vol. 40, no. 6, 1 June 2007 (2007-06-01), page S47, XP022121665, ISSN: 8756-3282
- ASSOULINE-DAYAN YEHUDITH ET AL: "Pathogenesis and natural history of osteonecrosis.", SEMINARS IN ARTHRITIS AND RHEUMATISM OCT 2002, vol. 32, no. 2, October 2002 (2002-10), pages 94-124, ISSN: 0049-0172
- LEE W Y ET AL: "The role of cytokines in the changes in bone turnover following bone marrow transplantation.", OSTEOPOROSIS INTERNATIONAL : A JOURNAL ESTABLISHED AS RESULT OF COOPERATION BETWEEN THE EUROPEAN FOUNDATION FOR OSTEOPOROSIS AND THE NATIONAL OSTEOPOROSIS FOUNDATION OF THE USA JAN 2002, vol. 13, no. 1, January 2002 (2002-01), pages 62-68, ISSN: 0937-941X
- OFOTOKUN IGHOVWERHA ET AL: "HIV-1 infection and antiretroviral therapies: risk factors for osteoporosis and bone fracture.", CURRENT OPINION IN ENDOCRINOLOGY, DIABETES, AND OBESITY DEC 2010, vol. 17, no. 6, December 2010 (2010-12), pages 523-529, ISSN: 1752-2978
- ZUPAN JANJA ET AL: "The relationship between osteoclastogenic and anti-osteoclastogenic pro-inflammatory cytokines differs in human osteoporotic and osteoarthritic bone tissues.", JOURNAL OF BIOMEDICAL SCIENCE 2012, vol. 19, 2012, page 28, ISSN: 1423-0127
- WEITZMANN M NEALE: "The Role of Inflammatory Cytokines, the RANKL/OPG Axis, and the Immunoskeletal Interface in Physiological Bone Turnover and Osteoporosis.", SCIENTIFICA 2013, vol. 2013, 2013, page 125705, ISSN: 2090-908X
- RHO JAERANG ET AL: "Osteoimmunology: interactions of the immune and skeletal systems.", MOLECULES AND CELLS 29 FEB 2004, vol. 17, no. 1, 29 February 2004 (2004-02-29), pages 1-9, ISSN: 1016-8478
- MORI GIORGIO ET AL: "The Interplay between the bone and the immune system.", CLINICAL & DEVELOPMENTAL IMMUNOLOGY 2013, vol. 2013, 2013, page 720504, ISSN: 1740-2530
- ROBERTO PACIFICI: "T cells, osteoblasts, and osteocytes: interacting lineages key for the bone anabolic and catabolic activities of parathyroid hormone", ANNALS OF THE NEW YORK ACADEMY OF SCIENCES, vol. 1364, no. 1, 10 January 2016 (2016-01-10), pages 11-24, US ISSN: 0077-8923, DOI: 10.1111/nyas.12969
- MOORE C ET AL: "Dendritic cells and B cells cooperate in the generation of CD4(+)CD25(+)FOXP3(+) allogeneic T cells.", TRANSPLANTATION PROCEEDINGS 2010 JAN-FEB, vol. 42, no. 1, January 2010 (2010-01), pages 371-375, ISSN: 1873-2623
- YASUHIRO KOBAYASHI ET AL: "Regulation of bone metabolism by Wnt signals", JOURNAL OF BIOCHEMISTRY, vol. 159, no. 4, 28 December 2015 (2015-12-28), pages 387-392, GB ISSN: 0021-924X, DOI: 10.1093/jb/mvv124
- ZAISS MARIO M ET AL: "Increased bone density and resistance to ovariectomy-induced bone loss in FoxP3-transgenic mice based on impaired osteoclast differentiation.", ARTHRITIS AND RHEUMATISM AUG 2010, vol. 62, no. 8, August 2010 (2010-08), pages 2328-2338, ISSN: 1529-0131

## Description

### Field of the Invention

The invention relates to therapeutic applications of bone-forming cells in the treatment of conditions and bone diseases associated with immunodeficiency or immunosuppression.

### Background to the Invention

Immunodeficiency disorders represent a diverse group of severe conditions in which compromised function of the immune system causes increased susceptibility of patients to various opportunistic infections or conditions.

Immunodeficiency can also result from administration of immunosuppressants (e.g., to prevent rejection of transplanted organs) or as a side effect of treatments directed to other pathologies, such as, e.g., chemotherapy.

There exists a need for further treatment modalities in immunodeficiency disorders.

Human mesenchymal stem cells (MSC) express high levels of human leukocyte antigen (HLA) class I and do not express HLA class II but can be induced to express the latter by stimulation with interferon γ (IFN γ) (Le Blanc et al. 2003. Exp Hematol 31: 890-6).

It has been reported that MSC could also act as antigen-presenting cells. This function was observed in a narrow time window and only after IFNγ administration and/or stimulation, but not on non-stimulated MSC. In presence of specific antigen (from *Candida albicans* or tetanus toxoid), MSC cells are able to up-regulate their HLA class II antigen expression by autocrine secretion of low IFNγ levels. However, when IFNγ concentration in culture increases, HLA class II expression is down-regulated and the APC function of MSC are inhibited (Stagg et al. 2006. Blood 107: 2570-7; Chan et al. 2006. Blood 107: 4817-24).

Osteoblasts from trabecular bone or from cell lines have been shown to be immunocompetent and to stimulate allogeneic peripheral blood mononuclear cells (Skjodt et al. 1989. Immunology 68: 416-20).

WO 2007/093431 relates to a method for osteogenic differentiation of bone marrow stem cells (BMSC) and uses thereof.

Kim Seok-Jung et al. (Journal of Medical Case Reports 2008, vol. 2, 58) concerns a case report of the treatment of osteonecrosis of the femoral head using autologous cultured osteoblasts.

Gangji et al (Bone, 2007, vol. 40, S47) concerns a case report of the treatment of osteonecrosis of the femoral head with implantation of autologous preosteoblastic cells into the necrotic zone.

### Description of the Invention

Unless otherwise defined, all terms used in disclosing the invention, including technical and scientific terms, have the meaning as commonly understood by one of ordinary skill in the art to which this invention belongs.

As used herein, the singular forms "a", "an", and "the" include both singular and plural referents unless the context clearly dictates otherwise. By way of example, "a cell" refers to one or more than one cell.

The terms "comprising", "comprises" and "comprised of" as used herein are synonymous with "including", "includes" or "containing", "contains", and are inclusive or open-ended and do not exclude additional, non-recited members, elements or method steps.

The recitation of numerical ranges by endpoints includes all numbers and fractions subsumed within that range, as well as the recited endpoints.

The term "about" as used herein when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" refers is itself also specifically, and preferably, disclosed.

All documents cited in the present specification are hereby incorporated by reference in their entirety. In particular, the teachings of all documents herein specifically referred to are incorporated by reference.

The present inventors surprisingly realised that bone-forming cells display potent antigen presenting cell (APC) properties, in addition to expected osteoregenerative actions, and are thus useful in the treatment of diseases and conditions associated with immunodeficiency or immunosuppression.

Advantageously, the inventors also found that bone-forming cells, such as in particular bone-forming cells obtainable *in vitro* by differentiation from isolated mesenchymal stem cells (MSC) or bone marrow stromal cells (BMSC), can display distinctive immunological markers (such as, e.g., HLA class II antigen) and antigen presenting cell properties substantially independently of activation or stimulation (such as, e.g., by interferon gamma). Said APC markers and properties are thus not necessarily confined to a narrow time window following an activation or stimulation. Such comparatively steady APC properties make bone-forming cells an advantageous agent in the treatment of the above diseases and conditions.

Accordingly, the present invention provides subject-matter as set forth in any one and all of (i) to (vii) below:
(i) Isolated bone-forming cells for use in treating a bone disease or condition associated with immunodeficiency or immunosuppression, wherein said bone-forming cells are osteoblasts or osteocytes and wherein the bone-forming cells comprise expression of HLA-II, and wherein said bone disease or condition associated with immunodeficiency or immunosuppression is osteoporosis, osteopenia, bone fragility, bone necrosis, bone fracture, bone microfractures, or bone osteolysis.
(ii) Isolated bone-forming cells for use as set forth in (i) above, wherein the bone-forming cells display the following characteristics:
   a) the cells comprise expression of alkaline phosphatase (ALP);
   b) the cells comprise expression of any one or more of procollagen type 1 amino-terminal propeptide (P1NP), osteonectin (ON), osteopontin (OP), osteocalcin (OCN) and bone sialoprotein (BSP);
   c) the cells show evidence of ability to mineralize the external surroundings, or synthesize calcium-containing extracellular matrix;
   d) the cells comprise expression of HLA-I and HLA-II.
(iii) Isolated bone-forming cells for use as set forth in (i) or (ii) above, wherein said bone-forming cells are obtainable by differentiation from mesenchymal stem cells (MSC) or bone marrow stromal cells (BMSC).
(iv) Isolated bone-forming cells for use as set forth in any one of (i) to (iii) above, wherein said bone-forming cells show evidence of antigen-presenting properties.
(v) Isolated bone-forming cells for use as set forth in any one of (i) to (iv) above, wherein the bone-forming cells are of human origin and the medicament is to be employed for autologous or allogeneic administration to human subjects.
(vi) A pharmaceutical composition comprising bone-forming cells comprising expression of HLA-II and a pro-inflammatory cytokine, for simultaneous, sequential or separate use in treating a bone disease or condition associated with immunodeficiency or immunosuppression, wherein said bone disease or condition associated with immunodeficiency or immunosuppression is osteoporosis, osteopenia, bone fragility, bone necrosis, bone fracture, bone microfractures, or bone osteolysis.
(vii) The pharmaceutical composition for use as set forth in (vi) above, wherein the pro-inflammatory cytokine is IFNγ, TNFα, IL-1β**,** IL-17 or IL-18.

The subject-matter provided by the invention thus specifically belongs to the disclosure, description and teaching of the present specification.

The specification further describes:
- isolated bone-forming cells for use in treating a disease or condition associated with immunodeficiency or immunosuppression;
- the use of isolated bone-forming cells for the manufacture of a medicament for the treatment of a disease or condition associated with immunodeficiency or immunosuppression;
- a method for preventing and/or treating a disease or condition associated with immunodeficiency or immunosuppression in a subject in need of such treatment, comprising administering to said subject a prophylactically or therapeutically effective amount of isolated bone-forming cells;
- a pharmaceutical composition comprising isolated bone-forming cells for use in treating a disease or condition associated with immunodeficiency or immunosuppression.

The term "isolating" with reference to a particular component denotes separating that component from at least one other component of a composition from which the former component is thereby "isolated". The term "isolated" as used herein in relation to cells or cell populations also implies that such cells or cell populations do not form part of an animal or human body, but are removed or separated there from, such as, e.g., maintained and/or propagated in cell culture.

The bone-forming cells may preferably be of animal origin, more preferably of mammal origin including non-human mammal origin and even more preferably of human origin.

The bone-forming cells may be usually obtained from or derived from a biological sample of a subject (i.e., a sample removed from a subject and comprising cells thereof) such as preferably a human or non-human mammal subject.

The bone-forming cells may be preferably employed for autologous administration (i.e., administered to the same subject from which the cells have been obtained or derived) or allogeneic administration (i.e., administered to a subject other than, but of the same species as, the subject from which the cells have been obtained or derived). Also possible may be xenogenic administration of said bone-forming cells (i.e., wherein cells obtained or derived from a subject of one species are administered to a subject of a different species).

Preferably herein, human bone-forming cells are to be employed for autologous or allogeneic administration to human subjects having a disease or condition associated with immunodeficiency or immunosuppression.

The term "bone-forming cells" as used herein generally refers to cells capable of contributing to the formation of bone material and/or bone matrix, and particularly denotes isolated cells or cell populations which have partly or fully progressed along osteogenic differentiation pathway. Without limitation, bone-forming cells particularly encompass osteoprogenitors, osteoblasts, osteocytes and other cell types of the osteogenic lineage as known in the art.

A skilled person thus generally appreciates the bounds of the term "bone-forming cells" as intended herein. Nevertheless, by means of further guidance and not limitation the present bone-forming cells may display any one, more or all following characteristics:
a) the cells comprise expression of alkaline phosphatase (ALP), more specifically ALP of the bone-liver-kidney type;
b) the cells comprise expression of any one or more or all of procollagen type 1 amino-terminal propeptide (P1NP), osteonectin (ON), osteopontin (OP), osteocalcin (OCN) and bone sialoprotein (BSP);
c) the cells show evidence of ability to mineralize the external surroundings, or synthesize calcium-containing extracellular matrix (e.g., when exposed to osteogenic medium; see Jaiswal et al. 1997. J Cell Biochem 64: 295-312). Calcium accumulation inside cells and deposition into matrix proteins can be conventionally measured for example by culturing in ⁴⁵Ca²⁺, washing and re-culturing, and then determining any radioactivity present inside the cell or deposited into the extracellular matrix (US 5,972,703), or using an Alizarin red-based mineralization assay (see, e.g., Gregory et al. 2004. Analytical Biochemistry 329: 77-84);
d) the cells substantially do not differentiate towards any one of, and preferably towards neither of cells of adipocytic lineage (e.g., adipocytes) or chondrocytic lineage (e.g., chondrocytes). The absence of differentiation towards such cell lineages may be tested using standard differentiation inducing conditions established in the art (e.g., see Pittenger et al. 1999. Science 284: 143-7), and assaying methods (e.g., when induced, adipocytes typically stain with oil red O showing lipid accumulation; chondrocytes typically stain with alcian blue or safranin O). Substantially lacking propensity towards adipogenic and/or chondrogenic differentiation may typically mean that less than 50%, or less than 30%, or less than 5%, or less than 1% of the tested cells would show signs of adipogenic or chondrogenic differentiation when applied to the respective test.

In an embodiment the bone-forming cells may display all characteristics listed under a), c) and d) above.

In an embodiment, the bone-forming cells are positive for (i.e., comprise expression of) HLA class II antigens.

In an embodiment, the bone-forming cells are positive for (i.e., comprise expression of) HLA class I and HLA class II antigens.

In a further embodiment, the bone-forming cells show evidence of antigen-presenting cell (APC) properties.

In another embodiment, the bone-forming cells may show evidence of immunosuppressive properties.

Where a cell is said to be positive for a particular component (e.g., marker or enzyme), this means that a skilled person will conclude the presence or evidence of a distinct signal, e.g., antibody-detectable or detection by reverse transcription polymerase chain reaction, for that component when carrying out the appropriate measurement, compared to suitable controls. Where the method allows for quantitative assessment of the component, positive cells may on average generate a signal that is significantly different from the control, e.g., but without limitation, at least 1.5-fold higher than such signal generated by control cells, e.g., at least 2-fold, at least 4-fold, at least 10-fold, at least 20-fold, at least 30-fold, at least 40-fold, at least 50-fold higher or even higher.

The expression of the above cell-specific markers can be detected using any suitable immunological technique known in the art, such as immuno-cytochemistry or affinity adsorption, Western blot analysis, FACS, ELISA, etc., or by any suitable biochemical assay of enzyme activity (e.g., for ALP), or by any suitable technique of measuring the quantity of the marker mRNA, e.g., Northern blot, semi-quantitative or quantitative RT-PCR, etc. Sequence data for markers listed in this disclosure are known and can be obtained from public databases such as GenBank (http://www.ncbi.nlm.nih.gov/).

Isolated bone-forming cells or cell populations for use in the invention or as described herein may be obtained or derived in any suitable manner known in the art. Without limitation, one suitable method to obtain bone-forming osteoblasts, more particularly HLA class II positive osteoblasts and osteoblast populations, has been disclosed in WO 2007/093431 and involves culturing isolated bone marrow stromal cells (BMSC) or mesenchymal stem cells (MSC) in the presence of serum or plasma and basic fibroblast growth factor (FGF-2). In another example, bone-forming osteoblasts, more particularly HLA class II positive osteoblasts, can be directly isolated and cultured from trabecular bone as described by Skjodt et al. 1985 (J Endocrinol 105: 391-6). In a yet further example, osteogenic lineage cells may be obtained by differentiating MSC in osteogenic medium as described by Pittenger et al. 1999 (Science 284: 143-7) and Jaiswal et al. 1997 (supra), preferably in the presence of FGF-2 to achieve HLA class II positive osteoblasts and osteoblast populations.

In a preferred embodiment, the isolated bone- forming cells for uses as defined herein, are obtainable or directly obtained by differentiation from BMSC or MSC. Advantageously, such differentiation may comprise exposing the BMSC or MSC to FGF-2 to achieve HLA class II positive osteoblasts and osteoblast populations. These cells display comparably stable APC properties which can be obtained without external activation factors.

The term "immunodeficiency" generally denotes a state when a subject's specific and/or nonspecific immune system function is pathologically reduced or absent. Diseases or conditions associated with immunodeficiency or "immunodeficiency disorders" refer to a diverse group of conditions characterised primarily by an increased susceptibility to various opportunistic infections with consequent severe acute, recurrent or chronic disease, resulting from immunodeficiency due to one or more defects in the immune system. Immunodeficiency disorders encompass, without limitation, "immunodeficiency syndromes" wherein all features are the result of the immune defect, and "syndromes with immunodeficiency", wherein some, even prominent features cannot be explained by the immune defect.

The group of immunodeficiency disorders also encompasses diseases and conditions associated with immunosuppression, where the latter term refers to artificial, usually controlled diminution or prevention of a subject's immune response. Immunosuppression in subjects may be caused by immunosuppressants ("immunosuppressant" refers to any compound or agent that is known or found to suppress or prevent an undesired immune response, such as, e.g., prevent the immune system's rejection of a transplanted organ; examples of immunosuppressants include, without limitation, cyclosporin A, mycophenolate mofetil, rapamycin, FK506 and corticosteroids), or it may occur as a side effect of a therapy of other indications (e.g., side effect of cancer chemotherapy).

By means of example and not limitation, diseases and conditions associated with immunodeficiency or immunosuppression comprise: human immunodeficiency virus (HIV) infection and acquired immune deficiency syndrome (AIDS), hypogammaglobulinemia, hematologic cancers such as leukaemia and lymphoma, total bone marrow ablation, bone marrow transplantation, organ transplantation, lymphocytopenia (lymphopenia) of any origin, lupus erythematosus, cachexia, opioids abuse, mastocytosis, rheumatic fever, trypanosomiasis, alcohol abuse; and treatments with: chemotherapy agents, corticosteroids, anti-TNF drugs, radiation, immunosuppressive drugs such as *inter alia* tacrolimus, cyclosporine, methotrexate, mycophenolate, azathioprine, interferons, and immunoglobulins such anti-CD 20 and anti-CD3.

In an embodiment, the diseases or conditions associated with immunodeficiency or immunosuppression may also include a bone-dysfunction or bone-lesion component (such as, e.g., osteoporosis, osteonecrosis, osteopenia, bone fragility, bone necrosis, bone fracture (or microfractures), bone sclerosis, and bone osteolysis). Advantageously, the bone-forming cells of the invention can synergically act on such diseases by ameliorating the immunodeficiency and stimulating bone-reconstruction.

Hence, the specification also describes:
- isolated bone-forming cells for use in treating a bone disease or condition associated with immunodeficiency or immunosuppression;
- the use of isolated bone-forming cells for the manufacture of a medicament for the treatment of a bone disease or condition associated with immunodeficiency or immunosuppression;
- a method for preventing and/or treating a bone disease or condition associated with immunodeficiency or immunosuppression in a subject in need of such treatment, comprising administering to said subject a prophylactically or therapeutically effective amount of isolated bone-forming cells;
- a pharmaceutical composition comprising isolated bone-forming cells for use in treating a bone disease or condition associated with immunodeficiency or immunosuppression.

Preferably, human bone-forming cells may be employed for autologous or allogeneic administration to human subjects having a bone disease or condition associated with immunodeficiency or immunosuppression.

The isolated bone-forming cells can be administered to subjects, for treating diseases or conditions associated with immunodeficiency or immunosuppression, including bone diseases or conditions associated with immunodeficiency or immunosuppression, as defined above.

Optionally, to stimulate the APC properties of the bone-forming cells, the cells may be treated with a pro-inflammatory cytokine prior to administration, or alternatively, may be administered in conjunction with (e.g., simultaneously or sequentially or separately in any order) a pro-inflammatory cytokine. Examples of pro-inflammatory cytokines include without limitation IFNγ, TNFα, IL-1β, IL-17, IL-18.

The terms "subject" or "patient" refer preferably to animals, more preferably warm-blooded animals, yet more preferably vertebrates, and even more preferably mammals specifically including humans and non-human mammals, that have been the object of treatment, observation or experiment. The term "mammal" includes any animal classified as such, including, but not limited to, humans, domestic and farm animals, zoo animals, sport animals, pet animals, companion animals and experimental animals, such as, for example, mice, rats, hamsters, rabbits, dogs, cats, guinea pigs, cattle, cows, sheep, horses, pigs and primates, e.g., monkeys and apes. Particularly preferred are human subjects, including both genders and all age categories thereof.

The present treatments are particularly to be given to subjects in need thereof, which phrase includes subjects that would benefit from treatment of a given condition, such as a disease or condition associated with immunodeficiency or immunosuppression, including bone diseases or conditions associated with immunodeficiency or immunosuppression. Such subjects may include, without limitation, those that have been diagnosed with said condition, those prone to develop said condition and/or those in whom said condition is to be prevented.

The terms "treat" or "treatment" encompass both the therapeutic treatment of an already developed disorder, such as the therapy of an already developed disease or condition associated with immunodeficiency or immunosuppression, including a bone disease or condition associated with immunodeficiency or immunosuppression, as well as prophylactic or preventative measures, wherein the aim is to prevent or lessen the chances of incidence of an undesired affliction, such as to prevent the chances of contraction and progression of a disease or condition associated with immunodeficiency or immunosuppression. Beneficial or desired clinical results may include, without limitation, alleviation of one or more symptoms or one or more biological markers, diminishment of extent of disease, stabilised (*i.e.,* not worsening) state of disease, delay or slowing of disease progression, amelioration or palliation of the disease state, and the like. "Treatment" can also mean prolonging survival as compared to expected survival if not receiving treatment.

The term "prophylactically effective amount" refers to an amount of an active compound or pharmaceutical agent that inhibits or delays in a subject the onset of a disorder as being sought by a researcher, veterinarian, medical doctor or other clinician. The term "therapeutically effective amount" as used herein, refers to an amount of active compound or pharmaceutical agent that elicits the biological or medicinal response in a subject that is being sought by a researcher, veterinarian, medical doctor or other clinician, which may include *inter alia* alleviation of the symptoms of the disease or disorder being treated. Methods are known in the art for determining therapeutically and prophylactically effective doses.

The treatment may employ autologous (*i.e.,* cells derived from the subject to be treated), allogeneic (*i.e.,* cells derived from subject(s) other than the subject to be treated, but belonging to the same species) or xenogenic (*i.e.,* cells derived from subject(s) belonging to species other than the subject to be treated) bone-forming cells and cell populations as defined herein.

Envisaged are in particular treatments of human subjects using human autologous or allogeneic bone-forming cells or cell populations as defined herein.

Suitably, the herein defined bone-forming cells and cell populations may be formulated into and administered as pharmaceutical compositions.

Pharmaceutical compositions will typically comprise the bone-forming cells or cell populations as the active ingredient, and one or more pharmaceutically acceptable carrier/excipient. As used herein, "carrier" or "excipient" includes any and all solvents, diluents, buffers (such as, *e.g.,* neutral buffered saline or phosphate buffered saline), solubilisers, colloids, dispersion media, vehicles, fillers, chelating agents (such as, *e.g.,* EDTA or glutathione), amino acids (such as, *e.g.,* glycine), proteins, disintegrants, binders, lubricants, wetting agents, emulsifiers, sweeteners, colorants, flavourings, aromatisers, thickeners, agents for achieving a depot effect, coatings, antifungal agents, preservatives, stabilisers, antioxidants, tonicity controlling agents, absorption delaying agents, and the like. The use of such media and agents for pharmaceutical active substances is well known in the art. Such materials should be non-toxic and should not interfere with the activity of the cells.

The precise nature of the carrier or excipient or other material will depend on the route of administration. For example, the composition may be in the form of a parenterally acceptable aqueous solution, which is pyrogen-free and has suitable pH, isotonicity and stability. For general principles in medicinal formulation, the reader is referred to Cell Therapy: Stem Cell Transplantation, Gene Therapy, and Cellular Immunotherapy, by G. Morstyn & W. Sheridan eds., Cambridge University Press, 1996; and Hematopoietic Stem Cell Therapy, E. D. Ball, J. Lister & P. Law, Churchill Livingstone, 2000.

Such pharmaceutical compositions may contain further components ensuring the viability of the cells therein. For example, the compositions may comprise a suitable buffer system (e.g., phosphate or carbonate buffer system) to achieve desirable pH, more usually near neutral pH, and may comprise sufficient salt to ensure isoosmotic conditions for the cells to prevent osmotic stress. For example, suitable solution for these purposes may be phosphate-buffered saline (PBS), sodium chloride solution, Ringer's Injection or Lactated Ringer's Injection, as known in the art. Further, the composition may comprise a carrier protein, e.g., albumin, which may increase the viability of the cells.

The pharmaceutical compositions may comprise further components useful in the repair of bone wounds and defects. For example, such components may include without limitation bone morphogenetic proteins, bone matrix (e.g., bone matrix produced in vitro by cells of the invention or by other methods), hydroxyapatite/tricalcium phosphate particles (HA/TCP), gelatine, poly-lactic acid, poly-lactic glycolic acid, hyaluronic acid, chitosan, poly-L-lysine, and collagen. For example, the osteoblastic cells may be combined with demineralised bone matrix (DBM) or other matrices to make the composite osteogenic (bone forming in it own right) as well as osteo-inductive. Similar methods using autologous bone marrow cells with allogeneic DBM have yielded good results (Connolly et al. 1995. Clin Orthop 313: 8-18).

The pharmaceutical composition can further include or be co-administered with a complementary bioactive factor such as a bone morphogenic protein, such as BMP-2, BMP-7 or BMP-4, or any other growth factor. Other potential accompanying components include inorganic sources of calcium or phosphate suitable for assisting bone regeneration (WO 00/07639). If desired, cell preparation can be administered on a carrier matrix or material to provide improved tissue regeneration. For example, the material can be a granular ceramic, or a biopolymer such as gelatine, collagen, osteonectin, fibrinogen, or osteocalcin. Porous matrices can be synthesized according to standard techniques (e.g., Mikos et al., Biomaterials 14: 323, 1993; Mikos et al., Polymer 35:1068, 1994; Cook et al., J. Biomed. Mater. Res. 35:513, 1997).

The pharmaceutical compositions can further include or be co-administered in combination with any pro-inflammatory cytokine, which can enhance the APC properties of the bone-forming cells. Hence, the invention also provides a pharmaceutical composition comprising bone-forming cells and a pro-inflammatory cytokine for simultaneous, sequential or separate use in treating diseases and conditions associated with immunodeficiency or immunosuppression, including bone diseases or conditions associated with immunodeficiency or immunosuppression. Examples of pro-inflammatory cytokines include without limitation IFNγ, TNFα, IL-1β, IL-17, IL-18.

Alternatively or in addition, the present cells may be stably or transiently transformed with a nucleic acid of interest prior to introduction to the subject. Nucleic acid sequences of interest include, but are not limited to those encoding gene products that enhance the growth, differentiation and/or mineralization of osteoblasts. For example, an expression system for BMP-2, can be introduced in a stable or transient fashion for the purpose of treating non-healing fractures or osteoporosis. Methods of cell transformation are known to those skilled in the art.

The specification also describes an arrangement comprising a surgical instrument for administration of a composition to a subject, such as for example systemically, topically or at a site of bone lesion, and further comprising the cells or cell populations as described herein, or a pharmaceutical composition comprising said cells or cell populations, wherein the arrangement is adapted for administration of the pharmaceutical composition for example systemically, topically or at the site of bone lesion. For example, a suitable surgical instrument may be capable of injecting a liquid composition comprising cells of the present invention, such as systemically, topically or at the site of bone lesion.

The cells or cell populations can be administered in a manner that permits them to graft or migrate to the intended tissue site and reconstitute or regenerate the functionally deficient area. For example, the cells may be administered at a site of musculoskeletal lesion. For example, osteogenesis can be facilitated in concordance with a surgical procedure to remodel tissue or insert a split, or a prosthetic device such as a hip replacement. In other circumstances, invasive surgery will not be required, and the composition can be administered by injection or (e.g., for repair of the vertebral column) using a guidable endoscope.

In an embodiment the pharmaceutical cell preparation as defined above may be administered in a form of liquid composition. In embodiments, the cells or pharmaceutical composition comprising such can be administered systemically, topically or at a site of lesion.

In another embodiment, the cells or cell populations may be transferred to and/or cultured on suitable substrate to provide for implants. The substrate on which the cells can be applied and cultured can be a metal, such as titanium, cobalt/chromium alloy or stainless steel, a bioactive surface such as a calcium phosphate, polymer surfaces such as polyethylene, and the like. Although less preferred, siliceous material such as glass ceramics, can also be used as a substrate. Most preferred are metals, such as titanium, and calcium phosphates, even though calcium phosphate is not an indispensable component of the substrate. The substrate may be porous or non-porous.

For example, cells that have proliferated, or that are being differentiated in culture dishes, can be transferred onto three-dimensional solid supports in order to cause them to multiply and/or continue the differentiation process by incubating the solid support in a liquid nutrient medium described herein, if necessary. Cells can be transferred onto a three-dimensional solid support, e.g. by impregnating said support with a liquid suspension containing said cells. The impregnated supports obtained in this way can be implanted in a human subject. Such impregnated supports can also be re-cultured by immersing them in a liquid culture medium, prior to being finally implanted.

The three-dimensional solid support needs to be biocompatible so as to enable it to be implanted in a human. It can be of any suitable shape such as a cylinder, a sphere, a plate, or a part of arbitrary shape. Of the materials suitable for the biocompatible three-dimensional solid support, particular mention can be made of calcium carbonate, and in particular aragonite, specifically in the form of coral skeleton, porous ceramics based on alumina, on zirconia, on tricalcium phosphate, and/or hydroxyapatite, imitation coral skeleton obtained by hydrothermal exchange enabling calcium carbonate to be transformed into hydroxyapatite, or else apatite-wollastonite glass ceramics, bioactive glass ceramics such as Bioglass(TM) glasses.

The above aspects and embodiments are further supported by the following non-limiting examples.

The examples demonstrate that bone-forming cells, displaying class-I and class II-HLA, naturally or induced, may have interesting immunoregulatory properties as it could modulate T cell proliferation while having high bone reconstructive capabilities. These bone-forming cells are characterised by high expression levels of mesenchymal and bone surface markers and high mineralization capacity underlying their bone biological potential.

They are characterized by the expression of HLA class I and HLA class II molecules at their surfaces and by an absence of costimulatory molecules. Moreover, the HLA class II molecule can be regulated by different immune-stimulatory molecules (such as, *inter alia,* IFNγ, TNFα, IL-1β).

These bone-forming cells are capable to downregulate the proliferative response of stimulated T cells on an autologous and allogeneic basis. This demonstrates that both autologous and allogeneic bone-forming cell products will be particularly useful for the treatment of immune-related diseases.

In addition, and surprisingly with respect to the immunosuppressive properties mentioned above, these bone-forming cells can also play a role of antigen-presenting cells (APC), and are therefore capable to stimulate proliferation of T cells and other inflammatory cells.

Moreover, a small randomized, reference-controlled, clinical trial was performed, which convincingly demonstrated the beneficial effects of these bone-forming cells in a severe condition associated with immunodeficiency or immunosuppression, namely in stage I or II osteonecrosis of the femoral head associated with chronic immunosuppression due to the administration of high oral doses of corticosteroids for organ transplantation, autoimmune disease, or severe asthma.

Together, the data suggest that the cells will be particularly useful for the treatment of diseases or conditions associated with immunodeficiency or immunosuppression, optionally wherein such diseases involve a bone-dysfunction or bone-lesion component, including bone diseases or conditions associated with immunodeficiency or immunosuppression.

### Brief Description of the Figures

**Figure 1** illustrates mineralization by HLA-II positive bone-forming cells.
**Figure 2** illustrates ALP expression by HLA-II positive bone-forming cells. Figure 2A shows HLA-DR staining of the cells. Figure 2B shows ALP staining of the cells. In each figure, upper panel shows control staining, middle panel shows HLA-DR or ALP staining respectively, and the lower panel shows an overlay.
**Figure 3** illustrates VAS evolution in CTRL vs. APC-OB-treated patients.
**Figure 4** illustrates WOMAC™ Index evolution in CTRL vs. APC-OB-treated patients.
**Figure 5** illustrates bone lesion survival analysis in CTRL vs. APC-OB-treated patients.
**Figure 6****:** Radiological score evolution in CTRL vs. APC-OB-treated patients.

### Example 1

*Phenotypic analysis.* Immunobiological cell surface markers of the cells were analyzed by flow cytometry. Bone-forming cells were incubated with the following labelled monoclonal antibodies: HLA-I, HLA-DR, CD80, CD86, CTLA-4, CD40L and CD28 for 15 min and then washed with PBS before being centrifuged and resuspended in 0.3ml PBS.

*Cell differentiation.* HLA class II positive osteoblasts as used in the examples were generated from mesenchymal stem cells (MSC) *in vitro* using a conventional osteogenic culture medium (including DMEM or α-MEM medium supplemented with 10% FCS or autologous or allogeneic serum or plasma, dexamethasone (10⁻⁸M), ascorbic acid (50 µg/ml), and β-glycerophosphate (10 mM)) supplemented with FGF-2 (10 ng/ml).

*Mineralization assay - Bone Marrow mesenchymal cell differentiation in osteogenic medium.* Cells from culture are recovered by incubation with trypsin or versene and plated at 60 to 120,000 cells/well in 6-wells plate in the expansion medium (12,500 cells/cm²). The next day, the medium is replaced by 2.5 ml osteogenic medium. The cells are cultured for 2, 3 or 4 weeks. The medium is replaced every 3-4 days. After 2 weeks of culture, cells were fixed in 3.7% formaldehyde/PBS and stained by alizarin red

### Media.

### Dexamethasone dilution:

Dex1 (5.10⁻⁴M): 2 µl dexamethasone stock (5.10⁻²M) + 198 µl αMEM
Dex2 (10⁻⁶M): 2 µl Dex1 (5.10⁻⁴M) + 998 µl αMEM

**Osteogenic medium (40 ml)**

| | Volume | Final conc. |
|---|---|---|
| αMEM | 31 ml | / |
| FCS | 6 ml | 15% |
| PenStrepGlu (100x) | 400 µl | 1x |
| Dexameth. (Dex2) | 400 µl | 10⁻⁸M |
| Ascorbic acid | 200 µl | 50 µg/ml |
| β-glycerophosphate | 2 ml | 10 mM |

*Proliferation assay.* 200.000 human T cells/ml from individual A (PBMCa) were plated in 96-well microtitre plate with irradiated human bone-forming cells from individual B (OBb) for 7 days in a total volume of 200µl and in presence or absence of PHA (or another stimulatory agent).

Alternatively, 200,000 human T cells/ml from an individual A (PBMCa) were plated in 96-well microtitre plate with irradiated human bone-forming cells from individual B (OBb) for 7 days in a total volume of 200µl and in presence or absence of PBMCb from individual b (or of PBMCc from an individual c).

The bone-forming cells were seeded at 100.000 cells/ml. The culture was incubated with 1µCi/ml 3H-thymidine for 18h of the culture period to measure T cells proliferation. Cells were washed twice with ice-cold PBS and twice with Ice-cold 5% trichloroacetic acid (TCA). Finally, cells were lysed by a solution containing 0.1N NaOH and 0.1% Triton-X100. The supernatant is harvested and mixed with scintillation liquid to be analyzed on a beta-counter.

### Example 2

### Cell phenotype

Flow cytometry showed that this bone forming cell population expressed mesenchymal markers (CD90, CD105 and CD73) at high levels. Haematopoietic surface molecule are weak (CD45, CD34) or absent (CD14, CD19). The cells expressed high level of HLA class I but surprisingly, also high level of HLA-class II molecules. However, all the costimulatory molecules tested (CD80, CD86, CD28, CTLA-4) are absent **(Table 1).**

Interestingly, as shown by Le Blanc et al 2003 (Exp Hematol 31: 890-6) for MSC cells, we observed that the presence of IFNγ (5ng/ml) in the culture medium for 24 to 48h, induced an increase of HLA class II expression **(Table 1).** Similar observations were done with TNFα, IL-1β, IL-17, IL-18 and other pro-inflammatory cytokines.

**Table 1: Phenotypic markers of bone-forming cells cultured in presence of IFNγ or not (percentage of cells deemed positive by FACS)**

| | without IFNγ | | | with IFNγ | | |
|---|---|---|---|---|---|---|
| | N | Mean | SD | N | Mean | SD |
| ALP | 17 | 67.9 | 16.6 | 8 | 75.2 | 13.1 |
| CD105 | 17 | 92.8 | 3.9 | 8 | 89.9 | 4.9 |
| CD90 | 12 | 95.7 | 4.0 | 8 | 94.8 | 1.9 |
| CD73 | 13 | 94.7 | 3.4 | 8 | 96 | 2.8 |
| | | | | | | |
| CD45 | 17 | 2.8 | 3.3 | 8 | 1.4 | 2.1 |
| CD14 | 12 | 3.2 | 3.9 | 8 | 2.1 | 1.7 |
| CD19 | 13 | 4.8 | 4.8 | 8 | 2.3 | 2.3 |
| | | | | | | |
| HLA-class II | 16 | 68.3 | 18.9 | 8 | 92.3 | 7.2 |
| HLA-class I | 16 | 93.8 | 3.0 | 8 | 96.1 | 4.5 |
| CD86 | 14 | 4.3 | 4.8 | 8 | 6.9 | 3.1 |
| CD28 | 14 | 3.6 | 3.5 | 8 | 4.2 | 2.1 |
| CD80 | 15 | 2.1 | 2.2 | 8 | 3.2 | 2.0 |
| CD40L | 14 | 2.3 | 2.1 | 8 | 1.0 | 1.9 |
| CTLA4 | 14 | 5.7 | 6.2 | 8 | 9.5 | 3.5 |

### Example 3

### Immunomodulation

The bone-forming cell population described above was checked for its ability to regulate the immune system in *in-vitro* conditions. The induction or proliferation of T cell (peripheral blood mononucleated cells-PBMC) in autologous or heterologous conditions was tested.

HLA-class II+ bone forming cells were isolated and/or expanded from patients suffering of immune-related bone diseases. The level of expression of their HLA class II marker by flow cytometry and used their cells (bone-forming cells and/or PBMC) for T cell proliferation assays.

No proliferative response of PBMC - from recipient a - was observed when co-culture with HLA-class II+ bone forming cells in either autologous - from donor a - or allogeneic - from donor b - conditions **(Table 2).** This demonstrated that HLA-class II+ bone forming cells were not recognized as "foreign" cells by peripheral blood mononuclear cells and therefore were not able to elicit an allogeneic response.

Moreover, when stimulated by a mitogenic activator, such as PHA, PBMC were significantly downregulated by bone-forming cells in either autologous - from donor a - or allogeneic - from donor b- conditions. The level of immunosuppression was correlated with the initial level of HLA class II expression **(Table 2).**

**Table 2: Inhibitory effects of osteoblasts on allogeneic T cells proliferation (value are presented % increase or decrease - number of PBMC at start set at 100%).**

| **Bone-forming cells** | | | | | |
|---|---|---|---|---|---|
| MSC n° | HLA II | PBMCa | PBMCa + OBb | PBMCa + PHA | PBMCa + PHA + OBb |
| 3 | 52.8% | 100% | 107% | 700% | 425% (61%)* |
| 1 | 64.2% | 100% | 89% | 912% | 500% (55%) |
| 2 | 85.1% | 100% | 100% | 640% | 240% (37%) |
| 4 | 87.3% | 100% | 104% | 479% | 187% (39%) |

| | | | | | |
|---|---|---|---|---|---|
| * % of PBMC-PHA levels | | | | | |

These effects were not modified by pre-treatment with pro-inflammatory cytokines

### Example 4

### Antigen Presenting Properties

The addition of a second pool of PBMC from another donor leads to an increased proliferation of the PBMC from the recipient suggesting a role of APC of HLA-class II+ bone-forming cells **(Table 3).** That observation is correlated also with the expression levels of HLA class II.

**Table 3: Proliferative effects of bone-forming cells on T cells proliferation (from a recipient) in presence of PBMC from another donor (value are presented % increase or decrease - number of PBMC at start set at 100%).**

| **Bone-forming cells** | | | | | | |
|---|---|---|---|---|---|---|
| MSC n° | HLA II | PBMCa | PBMCa + PBMCb | PBMCa + OBb + PBMCb | PBMCa + PHA + PBMCb | PBMCa + PHA + OBb + PBMCb |
| 6 | 52.8% | 4,200 | 171% | 262% | 1488% | 1697% |
| 5 | 64.2% | 3,400 | 238% | 341% | 1676% | 2329% |
| 8 | 75.1% | 4,000 | 126% | 327% | 1450% | 2225% |
| 7 | 88.4% | 4,800 | 202% | 418% | 1572% | 2789% |

Interestingly, the addition of certain specific cytokines such as IL2, IL3, IL4, IL11, IL12 or PBMC from the beginning of the cell culture, leads to an increased proliferation of the PBMC suggesting a role of APC of HLA-class II+ bone forming cells (Table 4). That observation is also correlated with the expression levels of HLA class II.

**Table 4: Proliferative effects of bone-forming cells on T cells proliferation (from a recipient) in presence of IL2 (value are presented % increase or decrease - number of PBMC at start set at 100%).**

| **Bone-forming cells** | | | | | | |
|---|---|---|---|---|---|---|
| MSC n° | HLA II | PBMCa | PBMCa + IL2 | PBMCa + OBb + IL2 | PBMCa + PHA + IL2 | PBMCa + PHA + OBb + IL2 |
| 11 | 49.8% | 100% | 152% | 272% | 1138% | 1492% |
| 17 | 66.2% | 100% | 207% | 348% | 1346% | 2029% |
| 12 | 79.1% | 100% | 141% | 321% | 1510% | 2259% |

These effects were not modified by pre-treatment with pro-inflammatory cytokines.

### Example 5

### Bone reconstructive properties

The level of cellular markers (bone and mesenchymal markers) or membrane markers were assessed by flow cytometry **(Table 1).** The bone (ALP) and mesenchymal (CD105, 73, 90) markers were highly expressed with levels at >65% and >95% respectively

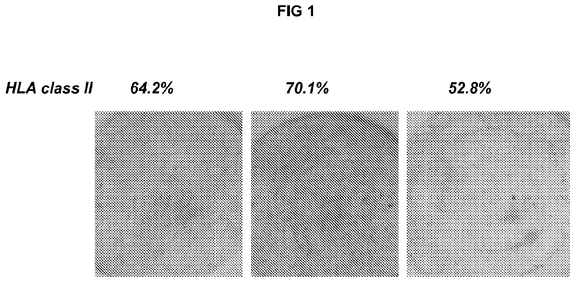

Correlation with bone biological function was done with ALP production (ALP staining) and the level of expression of the HLA class II marker (mineralization, **Figure 1**) suggesting that cells expressing high level of HLA class II molecules are capable of a high bone reconstructive potential.

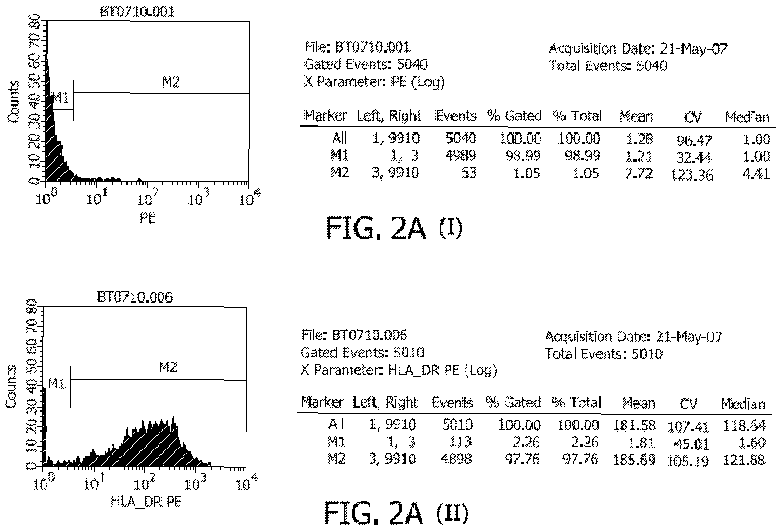

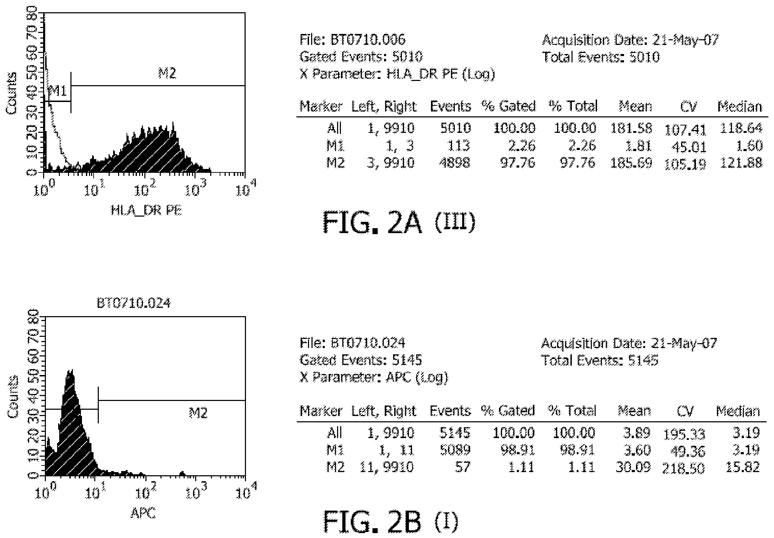

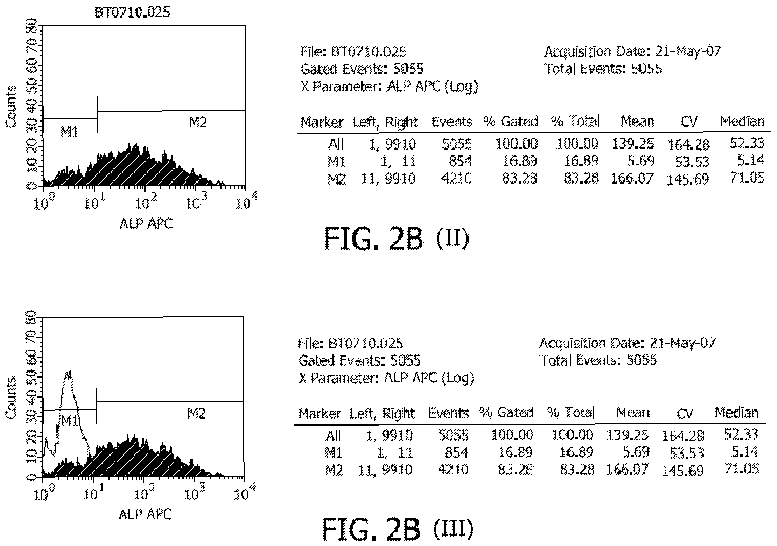

ALP was expressed by most, if not all cells (in cells incubated with IFNγ) **(****Figure 2****).**

### Example 6

### Human clinical efficacy data

### Design of the Study and Patient Populations

A small randomized, reference-controlled, clinical trial was performed, wherein eight patients with a severe condition associated with immunodeficiency or immunosuppression (*i.e.,* stage I or II osteonecrosis of the femoral head) were treated by core decompression associated with lesional implantation of either bone-forming cells presenting APC characteristics as obtained above (APC-OB, patients #4 to 8) or a population of bone marrow-derived mesenchymal stromal cells (control treatment, CTRL, patients #1 to 4).

In these patients, chronic immunosuppression was due to the administration of high oral doses of corticosteroids for organ transplantation, autoimmune disease, or severe asthma.

Treatment efficacy was investigated using both clinical (hip pain and function, as measured using visual analogue scale - VAS and WOMAC™ Index, respectively) and radiological (X-ray and MRI evidence of progression to fractural stages III or IV, according to the ARCO Classification of Osteonecrosis) criteria and endpoints. Patients were systematically evaluated and followed-up for 24 months.

### Study Results

### 1. Clinical Symptoms

### 1.1 Pain

Overall, a decrease in pain was observed in the APC-OB treated group after 12 to 24 months as compared to the CTRL group.

In the APC-OB-treated group, mean pain scores, as assessed by VAS, decreased from 45.5 (± 23) at baseline to 16.3 (± 14) at three months, 17.8 (± 15) at six months, 15 (± 11) at twelve months, 16.8 (± 11) at eighteen months, and 7.8 (± 12) at twenty-four months. By contrast, an increase in mean pain scores was found in the CTRL group, from 49 (± 32) at baseline to 60.5 (± 30.7) and 58.5 (± 18) at eighteen and twenty-four months, respectively).

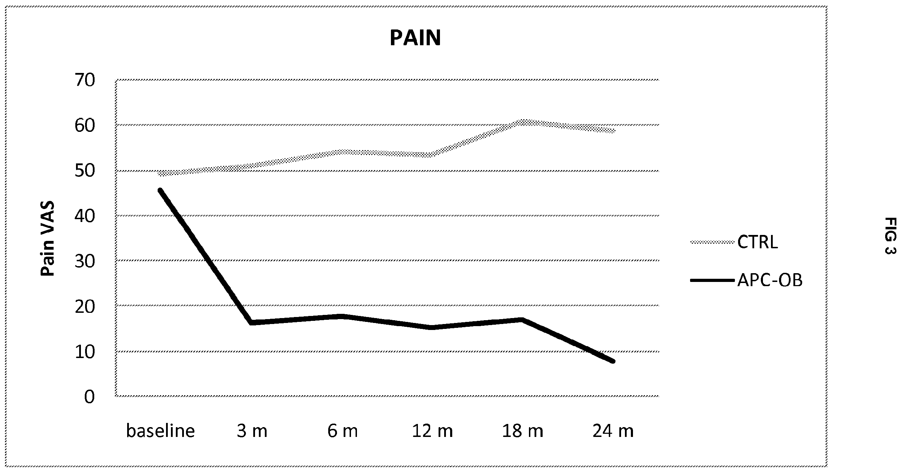

Over time, the benefits of treatment with APC-OB translates in differences between the two groups at twenty-four months of over 100% (see Figure 3).

### 1.2. Functional Scores

Similarly, in the APC-OB-treated group, mean WOMAC™ Index fell from 48 (± 25) at baseline to 17.8 (± 23) at three months, 20.5 (± 24) at six months, 15.5 (± 12) at twelve months, 18 (± 11) at eighteen months, and 18 (± 8) at twenty-four months. Conversely, WOMAC™ Index deteriorated in the CTRL groups over the same time period, from 39.8 (± 25) at baseline to 56.8 (± 22) and 57 (± 26) at eighteen and twenty-four months, respectively.

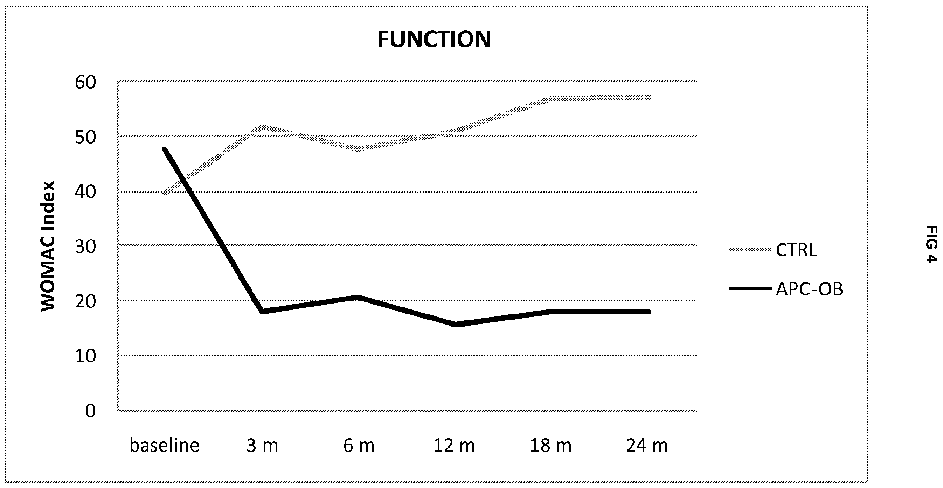

Over time, the benefits of treatment with APC-OB translates in differences between the two groups at twenty-four months of over 100% (see Figure 4).

### 1.3 Radiological Progression

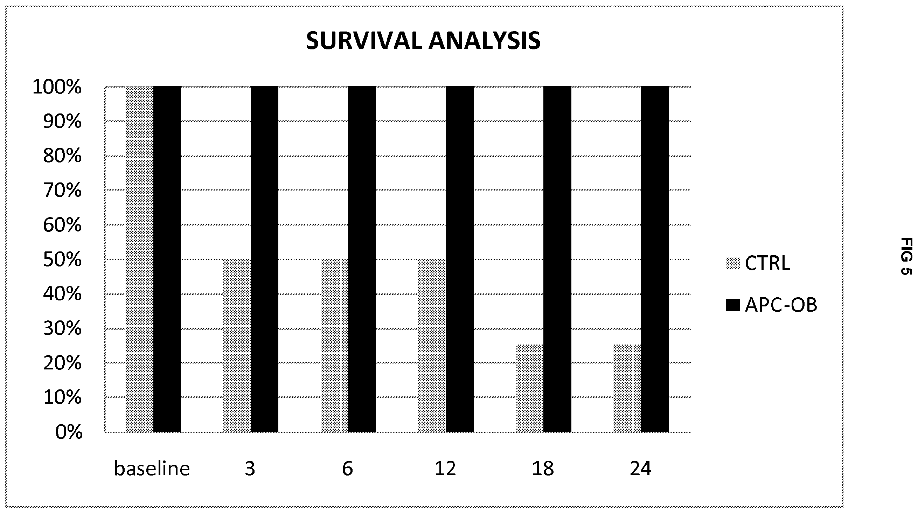

At 24 months, survival analysis demonstrated that 75% of bone lesions in the CTRL group had deteriorated to fracture against none in the APC-OB-treated group (Figure 5).

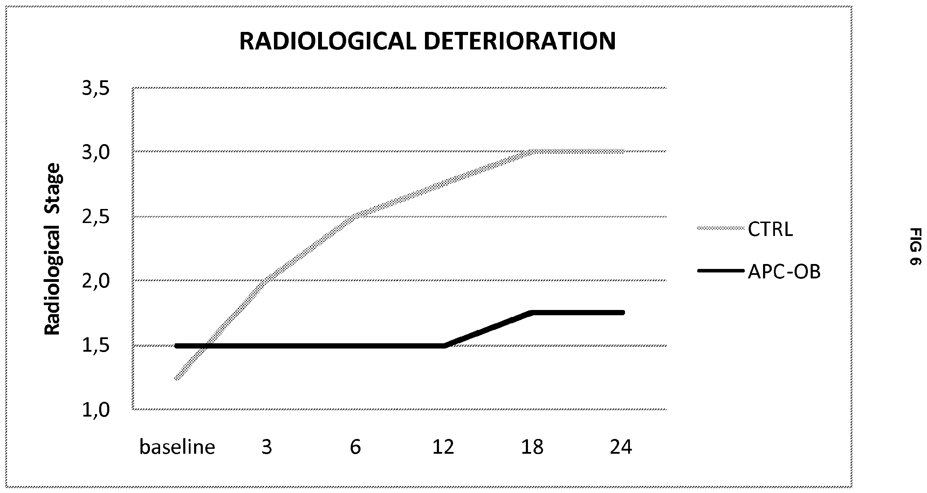

Moreover, as shown in Figure 6 illustrating mean radiological score evolution in the two patient groups, in the CTRL group the mean radiological score deteriorated from 1.3 at baseline to 2 at three months, 2.5 at six months, 2.8 at twelve months, and 3 at eighteen months and twenty four months. In comparison, APC-OB-treated patients only showed a minimal increase in mean radiological scores, from 1.5 at baseline to 1.5, 1.8 and 1.8 at twelve, eighteen, and twenty four months, respectively.

### Conclusions

In conclusion, our data indicates that patients (with stage I or II osteonecrosis of the femoral head related to corticoid-induced immunosuppression) receiving treatment with bone-forming cells presenting APC characteristics showed marked improvement of clinical and radiological endpoints, as compared to the control group.

## Claims

1. Isolated bone-forming cells for use in treating a bone disease or condition associated with immunodeficiency or immunosuppression, wherein said bone-forming cells are osteoblasts or osteocytes and wherein the bone-forming cells comprise expression of HLA-II, and wherein said bone disease or condition associated with immunodeficiency or immunosuppression is osteoporosis, osteopenia, bone fragility, bone necrosis, bone fracture, bone microfractures, or bone osteolysis.

2. Isolated bone-forming cells for use according to claim 1, wherein the bone-forming cells display the following characteristics:
a) the cells comprise expression of alkaline phosphatase (ALP);
b) the cells comprise expression of any one or more of procollagen type 1 amino-terminal propeptide (P1NP), osteonectin (ON), osteopontin (OP), osteocalcin (OCN) and bone sialoprotein (BSP);
c) the cells show evidence of ability to mineralize the external surroundings, or synthesize calcium-containing extracellular matrix;
d) the cells comprise expression of HLA-I and HLA-II.

3. Isolated bone-forming cells for use according to claim 1 or 2, wherein said bone-forming cells are obtainable by differentiation from mesenchymal stem cells (MSC) or bone marrow stromal cells (BMSC).

4. Isolated bone-forming cells for use according to any of claims 1 to 3, wherein said bone-forming cells show evidence of antigen-presenting properties.

5. Isolated bone-forming cells for use according to any of claims 1 to 4, wherein the bone-forming cells are of human origin and the medicament is to be employed for autologous or allogeneic administration to human subjects.

6. A pharmaceutical composition comprising bone-forming cells comprising expression of HLA-II and a pro-inflammatory cytokine, for simultaneous, sequential or separate use in treating a bone disease or condition associated with immunodeficiency or immunosuppression, wherein said bone disease or condition associated with immunodeficiency or immunosuppression is osteoporosis, osteopenia, bone fragility, bone necrosis, bone fracture, bone microfractures, or bone osteolysis.

7. The pharmaceutical composition for use according to claim 6, wherein the pro-inflammatory cytokine is IFNγ, TNFα, IL-1β, IL-17 or IL-18.

## Patentansprüche

1. Isolierte knochenbildende Zellen zur Verwendung in der Behandlung einer Knochenerkrankung oder eines Knochenleidens, welche(s) mit Immunschwäche oder Immunsuppression zusammenhängt, wobei die knochenbildenden Zellen Osteoblasten oder Osteozyten sind, und wobei die knochenbildenden Zellen Expression von HLA-II aufweisen, und wobei die Knochenerkrankung oder das Knochenleiden, die bzw. das mit Immunschwäche oder Immunsuppression zusammenhängt, Osteoporose, Osteopenie, Knochenbrüchigkeit, Knochennekrose, Knochenbruch, Knochenmikrofrakturen oder Knochenosteolyse ist.

2. Isolierte knochenbildende Zellen zur Verwendung nach Anspruch 1, wobei die knochenbildenden Zellen die folgenden Charakteristika aufweisen:
a) die Zellen weisen Expression von alkalischer Phosphatase (ALP) auf;
b) die Zellen weisen Expression von einem oder mehreren von aminoterminalem Propeptid des Prokollagens Typ 1 (P1NP), Osteonectin (ON), Osteopontin (OP), Osteocalcin (OCN) und Knochensialoprotein (BSP) auf;
c) die Zellen zeigen Belege für die Fähigkeit, die äußeren Umgebungen zu mineralisieren oder Calcium enthaltende extrazelluläre Matrix zu synthetisieren;
d) die Zellen weisen Expression von HLA-I und HLA-II auf.

3. Isolierte knochenbildende Zellen zur Verwendung nach Anspruch 1 oder 2, wobei die knochenbildenden Zellen durch Differenzierung aus mesenchymalen Stammzellen (MSC) oder Knochenmark-Stromazellen (BMSC) erhältlich sind.

4. Isolierte knochenbildende Zellen zur Verwendung nach einem der Ansprüche 1 bis 3, wobei die knochenbildenden Zellen Belege für Antigen-präsentierende Eigenschaften zeigen.

5. Isolierte knochenbildende Zellen zur Verwendung nach einem der Ansprüche 1 bis 4, wobei die knochenbildenden Zellen menschlichen Ursprungs sind und das Medikament zur autologen oder allogenen Verabreichung an menschliche Subjekte verwendet werden soll.

6. Pharmazeutische Zusammensetzung, umfassend knochenbildende Zellen, die Expression von HLA-II und proinflammatorischem Zytokin aufweisen, zur simultanen, sequentiellen oder separaten Verwendung in der Behandlung einer Knochenerkrankung oder eines Knochenleidens, welche(s) mit Immunschwäche oder Immunsuppression zusammenhängt, wobei die Knochenerkrankung oder das Knochenleiden, die bzw. das mit Immunschwäche oder Immunsuppression zusammenhängt, Osteoporose, Osteopenie, Knochenbrüchigkeit, Knochennekrose, Knochenbruch, Knochenmikrofrakturen oder Knochenosteolyse ist.

7. Pharmazeutische Zusammensetzung zur Verwendung nach Anspruch 6, wobei das proinflammatorische Zytokin IFNγ, TNFα, IL-1β, IL-17 oder IL-18 ist.

## Revendications

1. Cellules isolées de formation osseuse pour utilisation dans le traitement d'une maladie ou d'un état pathologique osseux associés à une immunodéficience ou une immunosuppression, où lesdites cellules de formation osseuse sont des ostéoblastes ou des ostéocytes, et où les cellules de formation osseuse comprennent l'expression de HLA-II, et où ladite maladie ou ledit état pathologique osseux associés à l'immunodéficience ou l'immunosuppression sont l'un des suivants : ostéoporose, ostéopénie, fragilité osseuse, nécrose osseuse, fracture osseuse, microfractures osseuses ou ostéolyse osseuse.

2. Cellules isolées de formation osseuse pour utilisation selon la revendication 1, où les cellules de formation osseuse présentent les caractéristiques suivantes :
a) les cellules comprennent l'expression de l'alcaline phosphatase (ALP) ;
b) les cellules comprennent l'expression d'un ou de plusieurs des suivants : propeptide N-terminal du procollagène de type 1 (P1NP), ostéonectine (ON), ostéopontine (OP), ostéocalcine (OCN) et sialoprotéine osseuse (BSP) ;
c) les cellules présentent des preuves d'une capacité à minéraliser leur environnement externe, ou à synthétiser une matrice extracellulaire contenant du calcium ;
d) les cellules comprennent l'expression de HLA-I et de HLA-II.

3. Cellules de formation osseuse isolées pour utilisation selon la revendication 1 ou 2, où lesdites cellules de formation osseuse peuvent être obtenues par différentiation à partir de cellules souches mésenchymateuses (CSM) ou de cellules stromales de moelle osseuse (CSMO).

4. Cellules isolées de formation osseuse pour utilisation selon l'une quelconque des revendications 1 à 3, où lesdites cellules de formation osseuse présentent des preuves de propriétés de présentation de l'antigène.

5. Cellules isolées de formation osseuse pour utilisation selon l'une quelconque des revendications 1 à 4, où les cellules de formation osseuse sont d'origine humaine et le médicament doit être employé pour administration autologue ou allogène à des sujets humains.

6. Composition pharmaceutique comprenant des cellules de formation osseuse comprenant l'expression de HLA-II et d'une cytokine pro-inflammatoire, pour utilisation simultanée, séquentielle ou séparée dans le traitement d'une maladie ou d'un état pathologique osseux associés à l'immunodéficience ou l'immunosuppression, où ladite maladie ou ledit état pathologique osseux associés à l'immunodéficience ou l'immunosuppression sont choisis parmi les suivants : ostéoporose, ostéopénie, fragilité osseuse, nécrose osseuse, fracture osseuse, microfractures osseuses ou ostéolyse.

7. Composition pharmaceutique pour utilisation selon la revendication 6, où la cytokine pro-inflammatoire est IFNγ, TNFα, IL-1β, IL-17 ou IL-18.
